# EUROPEAN PATENT APPLICATION

(11) **EP 2 712 559 A2**
(43) Date of publication of application: **02.04.2014**
(21) Application number: 13186163.5
(22) Date of filing: 26.09.2013
(51) Int. Cl.: A61B 17/3203, A61B 17/3207, A61B 17/00, A61B 17/22

(54) **Occlusion treatment system**

(30) Priority: 28.09.2012 US 201213630475
(71) Applicant: Covidien LP, Mansfield, MA 02048 (US)
(72) Inventor: Malhi, Arnaz, Watertown, MA Massachusetts 02472 (US)
(74) Representative: Gray, James

(57) **Abstract**

An occlusion treatment devices and methods are disclosed for treating a body lumen that is at least partially blocked by an occlusion. The occlusion treatment device includes an outer catheter with at least one expandable member, and an inner catheter with a cutting tip to debulk the occlusion. The inner catheter is axially movable through the outer catheter for repositioning between a retracted position, wherein the cutting tip is disposed within a passageway defined by outer catheter, and an advanced position, wherein at least a portion of the cutting tip is exposed from the outer catheter. During use of the occlusion treatment device, the transverse (radial) position of the cutting tip may be altered within the body lumen such that a distance defined between the cutting tip and an inner wall of the body lumen can be controllably varied by a user during a surgical procedure.

## Description

### TECHNICAL FIELD

The present disclosure relates to treatment of an occluded body lumen, specifically adjustable treatment of a body lumen.

### BACKGROUND

Various apparatus and methods for establishing and/or maintaining patency in a body lumen are known in the art. For example, thrombectomy catheter systems including a cutting tip are often used to debulk an occlusion, such as a chronic thrombus.

Known thrombectomy catheters treat occlusions in the immediate vicinity of their cutting tips. However, the transverse (radial) position of the cutting tips of these known thrombectomy catheters is not amenable to alteration. For example, transverse (radial) movement of the cutting tip *in situ* can result in contact between the cutting tip and an inner wall of the body lumen.

### SUMMARY

In one aspect of the present disclosure, an occlusion treatment device is disclosed that is configured and dimensioned to treat a body lumen that is at least partially blocked by an occlusion. The occlusion treatment device defines a central longitudinal axis, and includes an outer catheter with a proximal end portion and a distal end portion, as well as an inner catheter having a body portion with a proximal end portion and a distal end portion.

The outer catheter defines a passageway extending longitudinally therethrough. At least one expandable member is secured to an outer surface of the outer catheter.

The inner catheter includes a cutting tip adjacent the distal end portion of the body portion. The inner catheter is axially movable within the passageway between a retracted position, in which the inner catheter is stressed to assume a substantially linear orientation within the passageway, and an advanced position, in which at least a portion of the cutting tip is exposed (e.g., positioned distally beyond the outer catheter), and the inner catheter assumes a non-linear orientation. Advancement and retraction of the inner catheter relative to the outer catheter transversely (radially) moves the cutting tip relative to the central longitudinal axis defined by the occlusion treatment device such that, for example, a distance defined between the cutting tip and an inner wall of the body lumen can be controllably varied by a user.

The outer catheter may include first and second expandable members independently expandable relative to one another, whereby the distal end of the outer catheter can be transversely (radially) repositioned relative to the central longitudinal axis defined by the occlusion treatment device by varying pressure within the first and second expandable members.

The first and second expandable members may be positioned equidistant from the distal end of the outer catheter, or alternatively, the first expandable member may be positioned a first distance from the distal end of the outer catheter, and the second expandable member may be positioned a second, different distance from the distal end of the outer catheter.

The outer catheter may further include a third expandable member independently expandable of at least one of the first and second expandable members.

In some embodiments, the inner catheter may further include one or more sensors, e.g., a piezoelectric transducer, a magnetorestrictive transducer, or an optical element, oriented to transmit a signal transversely in relation to an outer sidewall of the inner catheter. For example, the inner catheter may include a sensor that is oriented to emit a signal orthogonally in relation to the outer sidewall of the inner catheter, e.g., in order to measure a distance from the sensor to the inner wall of the body lumen, and/or a sensor that is oriented to emit a signal subtending an angle less than 90° with the outer sidewall of the inner catheter, e.g., to measure a distance from the sensor to the inner wall of the body lumen and/or a distance to the occlusion.

The inner catheter may also include a sensor oriented to transmit a signal in orthogonal relation to an end wall of the inner catheter in order to measure an axial distance from the sensor to the occlusion.

In another aspect of the present disclosure, a method is disclosed for treating a body lumen that is at least partially blocked by an occlusion. The method includes: (i) positioning an occlusion treatment device within a body lumen, the occlusion treatment device including an outer catheter and first and second expandable members; (ii) advancing the outer catheter through the lumen until a distal end of the outer catheter is adjacent the occlusion; (iii) expanding first and second expandable members; (iv) advancing an inner catheter through the outer catheter lumen until a cutting tip adjacent a distal end portion of the inner catheter is adjacent the occlusion; (v) debulking the occlusion with the cutting tip; and (vi) moving the cutting tip within the body lumen transversely (radially) relative to a central longitudinal axis defined by the occlusion treatment device. Such transverse movement of the cutting tip relative to the central longitudinal axis can, for example, enlarge a treatment zone of the cutting tip.

Moving the cutting tip may include varying the pressure within the first and second expandable members. For example, moving the cutting tip may include varying the pressure within the first expandable member while the pressure within the second expandable member remains constant.

Additionally, or alternatively, moving the cutting tip may include advancing the inner catheter through the outer catheter such that a bent portion of the inner catheter is moved in relation to the distal end portion of the outer catheter to approximate the cutting tip and an inner wall of the body lumen.

The method may further include monitoring a distance defined between the cutting tip and the inner wall of the body lumen via one or more sensors that are secured to the inner catheter.

Embodiments can include one or more of the following advantages.

In some embodiments, an inner catheter is axially movable within a passageway defined by an outer catheter between a retracted position, in which the inner catheter is stressed to assume a substantially linear orientation within the passageway, and an advanced position, in which at least a portion of the cutting tip is exposed, and the inner catheter assumes a non-linear orientation. Such advancement and retraction of the inner catheter relative to the outer catheter transversely controllably moves the cutting tip relative to the central longitudinal axis. Such controlled movements can facilitate control over the position of the cutting tip, to increase the amount of material removed from occlusion, and/or treatment outcome, as compared to devices that do not allow controlled transverse movement within a body lumen. Additionally or alternatively, as compared to devices that do not allow controlled transverse movement within a body lumen, such control of transverse movement of the occlusion treatment device can facilitate debulking a vascular occlusion using a catheter that is much smaller in diametric size than the body lumen, further reducing the risk of perforating the vessel and potentially reducing trauma to the patient associated with larger diameter devices.

In certain embodiments, control of the transverse (radial) position of the cutting tip within the body lumen includes inflation and deflation of one or more expandable members. Such controlled movement can facilitate control over the position of the cutting tip, to increase the amount of material removed from occlusion, and/or treatment outcome, as compared to devices that do not allow controlled transverse movement within a body lumen.

Other aspects, features, and advantages will be apparent from the description and drawings, and from the claims.

### BRIEF DESCRIPTION OF THE DRAWINGS

FIG. 1 is a schematic of an occlusion treatment system including an occlusion treatment device.
FIG. 2 is a partial longitudinal, cross-sectional view of a central position of the occlusion treatment device seen in FIG. 1 within a body lumen blocked by an occlusion.
FIG. 3 is a transverse, cross-sectional view taken through lines 3-3 in FIG. 2.
FIG. 4 is a partial longitudinal, cross-sectional view of the occlusion treatment device seen in FIG. 1 positioned off-center within the body lumen.
FIG. 5 is a partial longitudinal, cross-sectional view of an occlusion treatment device within a body lumen blocked by an occlusion.
FIG. 6 is a transverse, cross-sectional view of an occlusion treatment device.
FIG. 7 is a partial longitudinal, cross-sectional view of a catheter.
FIG. 8 is a side, perspective view of a catheter.
FIG. 9 is a partial longitudinal, cross-sectional view of an occlusion treatment device positioned within a body lumen that is blocked by an occlusion.
FIG. 10 is a partial longitudinal, cross-sectional view of an occlusion treatment device positioned within a body lumen blocked by an occlusion.
FIG. 11 is a transverse, cross-sectional view taken through lines 11-11 in FIG. 10.
FIG. 12 is a partial longitudinal, cross-sectional view illustrating the occlusion treatment device seen in FIG. 10 in the context of a surgical procedure to treat a stent blocked by an occlusion.
FIG. 13 is an enlargement of the area of detail indicated in FIG. 12.
FIG. 14 is a schematic of a helical path followed by a catheter of the occlusion treatment device seen in FIG. 12 during rotation and deployment.
FIG. 15 is a schematic of steps in the procedure shown in FIG. 12.

### DETAILED DESCRIPTION

Embodiments of the present disclosure will now be described in detail with reference to the drawings, wherein like reference numerals identify similar or identical elements. As used herein, the term "distal" should be understood as referring to that portion of a device, or a component thereof, furthest from the user, such as a physician, during proper use, while the term "proximal" should be understood as referring to that portion of a device, or a component thereof, closest to the user during proper use. Additionally, the term "body lumen" should be understood to include any lumen within the body, either natural or artificial, such as, for example, blood vessels, blood vessel grafts, fistulas, and the like. Moreover, the term "occlusion" should be understood to encompass any partial or total blockage of a body lumen, such as, for example, a thrombus, an atheroma, plaque, or the like. Finally, as used herein, the terms "debulk" and "debulking," in the context of treating an occlusion, should be understood to include various methods of treating the occlusion, such as, for example, abrading, softening, melting, or otherwise breaking up the occlusion.

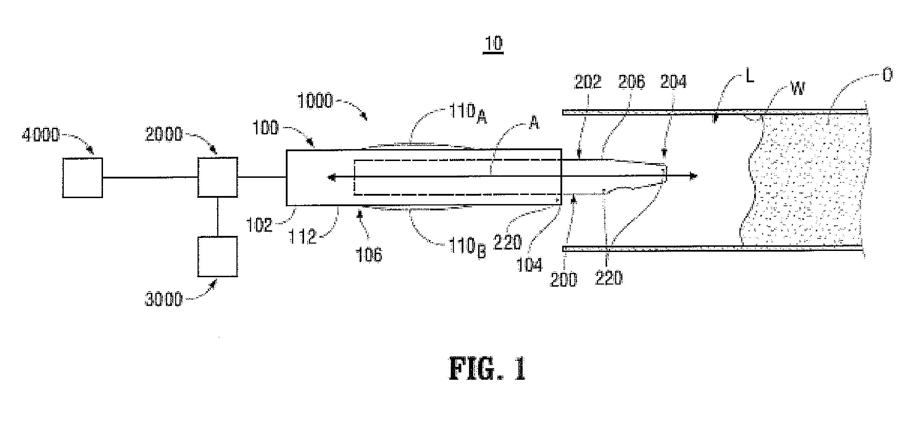

Referring now to FIG. 1, an occlusion treatment system 10 is used to treat a body lumen L, such as a blood vessel, that is at least partially blocked by an occlusion O. The occlusion treatment system 10 includes an occlusion treatment device 1000, a source of fluid 2000, a fluid suction/supply device 3000, and a control unit 4000.

The occlusion treatment device 1000 defines a central longitudinal axis A, and is insertable into the body lumen L to treat the occlusion O. The occlusion treatment device 1000 includes an outer catheter 100 and an inner catheter 200 movable through the outer catheter 100. The outer catheter 100 can be, for example, a guide catheter. Additionally or alternatively, the inner catheter 200 can be, for example, a thrombectomy catheter.

Referring now to FIGS. 1-4, the outer catheter 100 includes a proximal end portion 102 in communication with the control unit 4000, an open distal end portion 104, and a body portion 106 that extends between the respective proximal and distal end portions 102, 104. The body portion 106 defines a passage108 extending longitudinally therethrough. The passage 108 receives the inner catheter 200, and may be formed, such as by extrusion, from one or more biocompatible materials sufficiently pliable to facilitate insertion of the outer catheter 100 into the body lumen L. Suitable biocompatible materials of construction for the body portion 106 of the outer catheter 100 include, but are not limited to, one or more polymeric materials, elastomeric materials (for example, silicone and/or fabric materials), and/or synthetic resin (for example, polyurethane, polyethylene, polypropylene, nylons, polytetrafluoroethylene (PTFE), polyether ether ketone (PEEK), and/or polyimide).

The outer catheter 100 further includes expandable members 110_{A}, 110_{B} secured to an outer surface 112 of the outer catheter 100. The expandable members 110_{A}, 110_{B} may be formed from biocompatible material permitting selective expansion of the expandable members 110_{A}, 110_{B}, such as, for example, nylon, silicone, latex, polyolefin, vinyl, polyether block amide (e.g., PEBAX^{®}) polyethylene, polyurethane, and/or polyethylene terephthalate (PET).

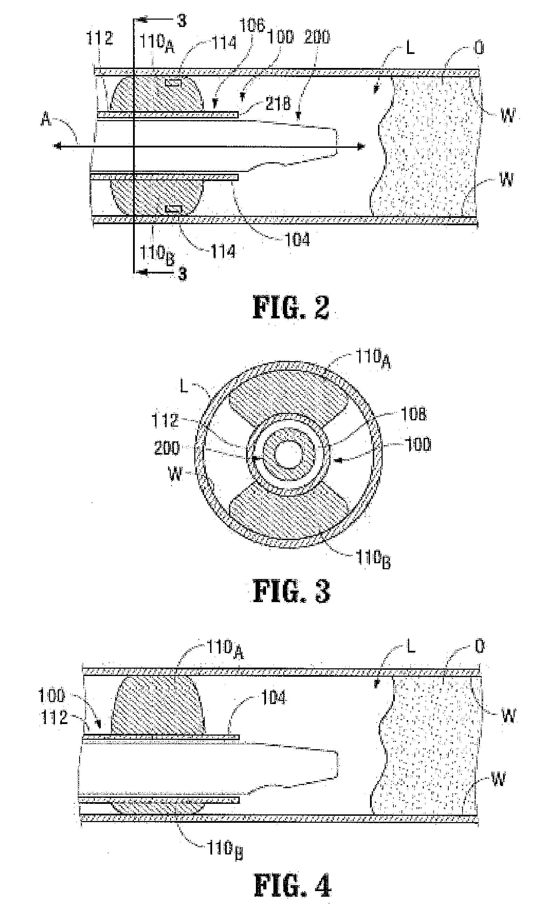

The expandable members 110_{A}, 110_{B} are each movable between an initial position, seen in FIG. 1, in which the expandable members 110_{A}, 110_{B} define first transverse cross-sectional dimensions. In a subsequent position, seen in FIGS. 2-4, the expandable members 110_{A}, 110_{B} define second transverse cross-sectional dimensions larger than the initial transverse cross-sectional dimensions shown in FIG. 1. In the initial position, the smaller transverse cross-sectional dimensions of the expandable members 110_{A}, 110_{B}, which may correspond to an outer transverse cross-sectional dimension of the body portion 106, facilitate insertion of the outer catheter 100 into the body lumen L, and movement of the outer catheter 100 through the body lumen L. In the subsequent position, the enlarged transverse cross-sectional dimensions of the expandable members 110_{A}, 110_{B} facilitate engagement of the expandable members 110_{A}, 110_{B} with an internal wall W of the body lumen L to facilitate maintaining the outer catheter 100 in a desired position within the body lumen L.

In some embodiments, the expandable members 110_{A}, 110_{B} are moved between the initial position and the subsequent position independently of each other. For example, the expandable members 110_{A}, 110_{B} can be moved from the initial position to the subsequent position via separate connections to the source of fluid 2000 (FIG. 1), and/or to the fluid suction/supply device 3000.

The expandable members 110_{A}, 110_{B} may be expanded to center the outer catheter 100 within the body lumen L, as shown, for example, in FIGS. 2-3. Centering the outer catheter 100 within the body lumen L can facilitate symmetrical spacing of the inner catheter 200 from the internal wall W of the body lumen L. Such symmetrical spacing of the inner catheter 200 from the internal wall W of the body lumen L can facilitate movement of a cutting tip 204 (FIG. 1) of the inner catheter 200, and control over the position of the cutting tip 204, to increase the amount of material removed from occlusion O, and/or treatment outcome. For example, the expandable members 110_{A}, 110_{B} may be expanded to space an outer surface 202 of the inner catheter 200 from the internal wall W of the body lumen L by a distance of about 0.5mm to about 2.0 mm .

The expandable members 110_{A}, 110_{B} are circumferentially spaced apart from one another on the outer surface 112 of the outer catheter 100. For example, the expandable members 110_{A}, 110_{B} may be positioned in diametrical opposition to one another. It should be appreciated, however, that the circumferential spacing of the expandable members 110_{A}, 110_{B} on the outer surface 112 of the outer catheter 100 may be altered in various embodiments, dependent upon the particular requirements of the surgical procedure in which the occlusion treatment system 10 is employed.

The expandable members 110_{A}, 110_{B} may be expanded to different extents. For example, the expandable members 110_{A}, 110_{B} may be formed from the same material, and the internal pressure of the expandable member 110_{A} may be different than the internal pressure of the expandable member 110_{B} such that the expandable members 110_{A}, 110_{B} are expanded by different radial distances from the outer surface 112 of the outer catheter 100. Additionally or alternatively, the internal pressures of the expandable members 110_{A}, 110_{B} may be approximately equal to one another, for example within ±10% of one another), and the expandable members 110_{A}, 110_{B} may be formed from different materials, such as, for example, materials with different coefficients of elasticity.

By varying the extent to which the expandable members 110_{A}, 110_{B} are radially expanded from the outer surface 112 of the catheter 100, the orientation of the distal end 104 of the outer catheter 100 can be adjusted within the body lumen L. For example, when the expandable members 110_{A}, 110_{B} are expanded equally, such as when the expandable members 110_{A}, 110_{B} are made of the same material and the internal pressure of the expandable member 110_{A} is equal to the internal pressure of the expandable member 110_{B}, the outer catheter 100 is positioned substantially centrally within the body lumen L. However, when the expandable members 110_{A}, 110_{B} are expanded to different extents, such as when the expandable members 110_{A}, 110_{B} are made of the same material and the internal pressures of the expandable members 110_{A}, 110_{B} are unequal, the outer catheter 100 can be positioned off-center within the body lumen L. For instance, when the expandable member 110_{A} is expanded to a greater extent than the expandable member 110_{B,} such as when the expandable members 110A, 110B are made of the same material and the internal pressure of the expandable member 110_{A} is caused to exceed the internal pressure of the expandable member 110_{B}, the distal end portion 104 of the outer catheter 100 is positioned closer to that portion of the inner wall W of the body lumen L adjacent the expandable member 110_{B}. When the expandable member 110_{B} is expanded to a greater extent than the expandable member 110_{A}, such as when the expandable members 110_{A}, 110_{B} are made of the same material and the internal pressure of the expandable member 110_{B} is caused to exceed the internal pressure of the expandable member 110_{A}, the distal end portion 104 of the outer catheter 100 is positioned closer to the portion of the inner wall W of the body lumen L adjacent the expandable member 110_{A}.

During use, following positioning of the outer catheter 100 within the body lumen L, expansion of the expandable members 110_{A}, 110_{B} can be maintained above a predetermined minimum threshold. For example, a minimal threshold pressure can be constantly maintained within each of the expandable members 110_{A}, 110_{B}, e.g., about 1 psi to about 5 psi. Maintaining expansion of the expandable members 110_{A}, 110_{B} above a predetermined minimum threshold can facilitate control over the position of the cutting tip 204 (FIG. 1) of the inner catheter 200 to increase the amount of material removed from occlusion O, and/or treatment outcome.

The outer catheter 100 may include one or more pressure sensors 114 that are associated with the expandable members 110_{A}, 110_{B}, as seen in FIG. 2 for example. The pressure sensors 114 can monitor the respective internal pressures of the expandable members 110_{A}, 110_{B}. The pressure sensors 114 may be in communication with the control unit 4000 such that respective internal pressures of the expandable members 110_{A}, 110_{B} may be controlled to achieve a desired effect. For example, the control unit 4000 may regulate expansion and contraction of the expandable members 110_{A}, 110_{B} to maintain a particular separation between the distal end portion 104 of the outer catheter 100 and the inner wall W of the body lumen L, or to vary the position of the inner catheter 200 within the body lumen L, and/or the position of the inner catheter 200 in relation to the occlusion O over time.

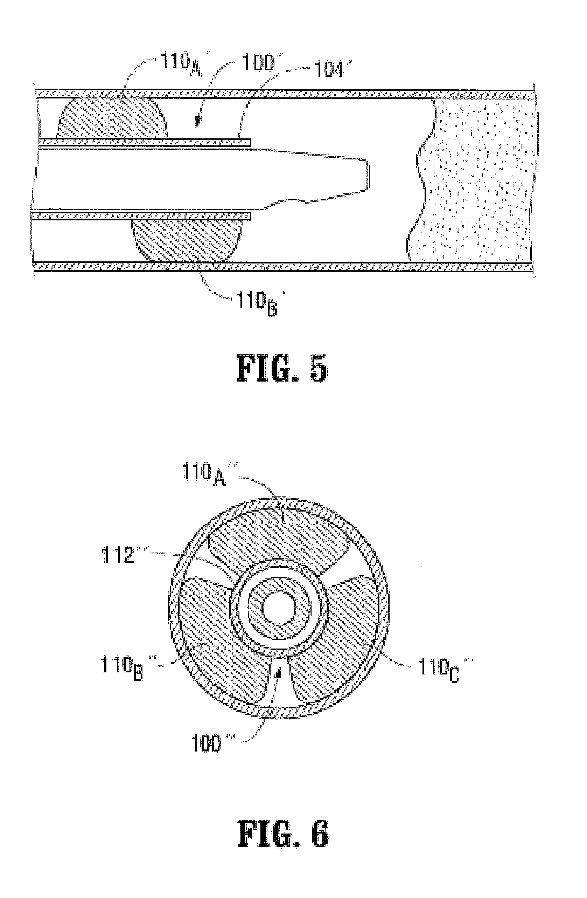

While expandable members have been described as positioned at the same axial location on an outer catheter such that the expandable members are spaced equidistant from a distal end portion of the outer catheter, other positions of the expandable members are additionally or alternatively possible. For example, as shown in FIG. 5, expandable members 110_{A'}, 110_{B'} may be positioned at different axial locations on an outer catheter 100' such that the distance defined between the expandable member 110_{A}' and the distal end portion 104' of the outer catheter 100 is different than the distance defined between the expandable member 110_{B} and the distal end 104 of the outer catheter 100.

While the outer catheter has been described as including two expandable members, other embodiments are additionally or alternatively possible. For example, referring to FIG. 6, an outer catheter 100" may include three expandable members 110_{A}", 110_{B}", 110_{C}", or more, e.g., four, five, six, etc.

Although shown as being spaced evenly from one another about the outer surface 112" of the outer catheter 100" in FIG. 6, the circumferential spacing of the expandable members 110_{A}", 110_{B}", 110_{C}" may be varied in some embodiments. For example, the expandable members 110_{A}", 110_{B}", 110_{C}" may be closer to each other than to the expandable member 110_{C}".

In certain embodiments, the expandable members 110_{A}", 110_{B}", 110_{C}" are expandable to different extents. For example, the expandable members 110_{A}, 110_{B}, 110_{C} may be expanded such that the internal pressure of the expandable member 110_{A} is different than the internal pressure of the expandable member 110_{B} and/or the internal pressure of the expandable member 110_{C}.

Although the expandable members 110_{A}", 110_{B}", 110_{C}" are shown as being positioned at the same axial location on the outer surface 112 of the outer catheter 100, one or more of the expandable members 110_{A}", 110_{B}", 110_{C}" may be positioned at different axial locations on outer surface 112 of the outer catheter 100.

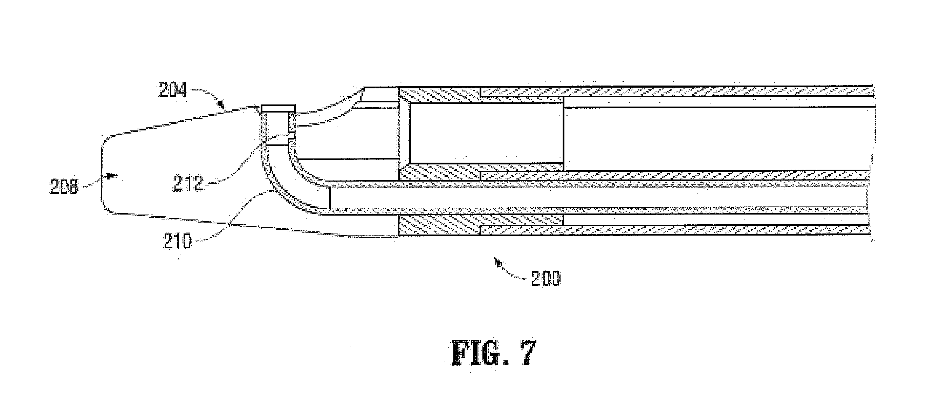

With reference now to FIGS. 1-4 and 7, the inner catheter 200 will be discussed. The inner catheter 200 includes a body portion 202, and the cutting tip 204, which is supported adjacent a distal end portion 206 of the body portion 202.

The body portion 202 of the inner catheter 200 may be formed, such as by extrusion, from a biocompatible material sufficiently pliable to facilitate insertion of the inner catheter 200 into the outer catheter 100 and into the body lumen L. Examples of biocompatible materials of construction for the body portion 202 of the inner catheter 200 include, but are not limited to, polymeric materials, elastomeric materials (e.g., silicone and fabric materials), and/or a synthetic resin (e.g., polyurethane, polyethylene, polypropylene, nylons, polytetrafluoroethylene (PTFE), polyether ether ketone (PEEK), or polyimide).

The body portion 202 of the inner catheter 200 may define an outer transverse cross-sectional dimension that substantially approximates an inner transverse cross-sectional dimension defined by the body portion 106 of the outer catheter 100. For example, it is envisioned that the body portion 202 of the inner catheter 200 may define an outer transverse cross-sectional dimension within the range of about 0.100 inches to about 0.110 inches, and the body portion 106 of the outer catheter 100 may define an inner transverse cross-sectional dimension within the range of about 0.115 inches to about 0.130 inches.

In some embodiments, the cutting tip 204 includes an atraumatic tip 208 carrying at least a portion of a high pressure tube 210. A nozzle 212 is in fluid communication with the high pressure tube 210 to create a fluid jet having sufficient pressure to debulk the occlusion O.

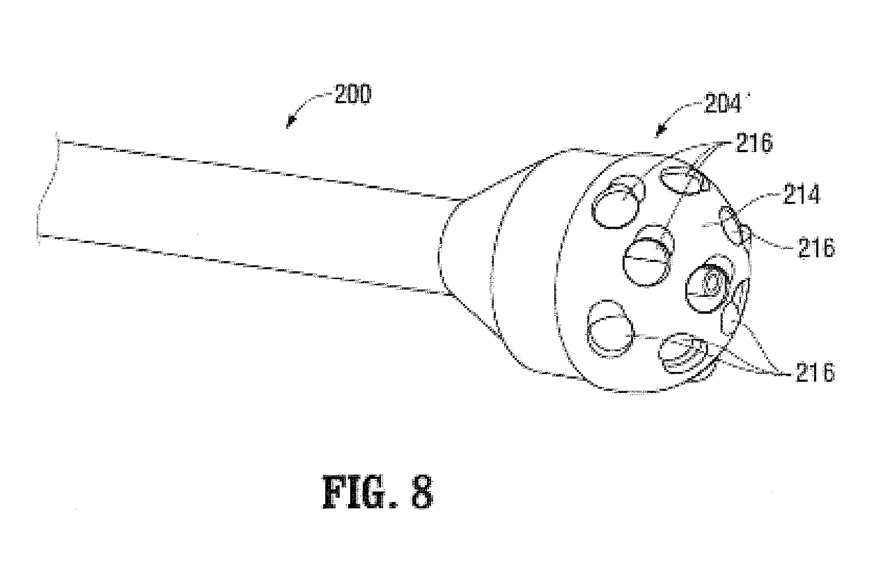

While the cutting tip has been described as having a single orifice nozzle, other cutting tip configurations are additionally or alternatively possible. For example, with reference to FIG. 8, a cutting tip 204' includes an outer surface 214 defining one or more apertures 216 formed therein that are configured and dimensioned to debulk the occlusion O (FIGS. 1, 2, 4).

As indicated above, the inner catheter 200 is movable through the outer catheter 100. Specifically, the inner catheter 200 is axially movable along the central longitudinal axis A of the occlusion treatment device 1000, such that the inner catheter 200 is movable between a retracted position, in which the cutting tip 204 is disposed within the outer catheter 100, and an advanced position, in which at least a portion of the cutting tip 204 is positioned distally beyond a distal end wall 218 (FIG. 2) of the body portion 106 of the outer catheter 100.

In some embodiments, the inner catheter 200 is rotatable relative to the outer catheter 100 to further facilitate the ability of the inner catheter 200 to debulk the occlusion O, as will be described in further detail below.

For example, the inner catheter 200 may be manually rotatable by the user. Additionally or alternatively, the inner catheter 200 may be operatively connected to a motor (not shown) within the control unit 4000. The rotational speed of the inner catheter 200 may be about 60 revolutions per minute (RPM) to about 200,000 RPM. For higher rotational speeds of the inner catheter 200, a torque coil (not shown) may be incorporated into the occlusion treatment device 1000.

Referring again to FIG. 1, the source of fluid 2000 maintains pressurized fluid, such as saline, therein. The source of fluid 2000 is in fluid communication with the occlusion treatment device 1000, and may include, for example, a pressurized tank, a pump, or the like.

The fluid suction/supply device 3000 is in fluid communication with the source of fluid 2000 such that fluid within the source of fluid 2000 can be moved to the occlusion treatment device 1000. The fluid suction/supply device 3000 may, additionally or alternatively, be in communication with the control unit 4000, for example, to regulate communication of fluid through the occlusion treatment device 1000.

The source of fluid 2000 and the fluid/suction supply device 3000 may be utilized, individually or in combination with one another, to communicate fluid to the occlusion treatment device 1000. For example, the source of fluid 2000 and the fluid/suction supply device 3000 may be utilized to expand the expandable members 110_{A}, 110_{B}. Additionally, or alternatively, the source of fluid 2000 and the fluid/suction supply device 3000 may be utilized to communicate fluid to, and withdraw fluid from, the treatment site of the occlusion O.

With continued reference to FIG. 1, the control unit 4000 regulates operation of the occlusion treatment device 1000 and may include various motors, microprocessors, electronic components, software, and/or firmware. Software may be provided in a machine-readable medium storing executable code, and/or other data, to facilitate the processing of user-specific input.

The control unit 4000 may provide the user with feedback from the occlusion treatment device 1000, and/or information pertaining to environmental conditions, operating parameters, etc. Additionally, it is envisioned that the control unit 4000 may output operational information concerning such feedback and information to the user. For example, the control unit 4000 may monitor the position of the cutting tip 204 of the inner catheter 200, the temperature at the site of the occlusion O, the volume of debris being withdrawn from the site of the occlusion O (discussed below), the elapsed time of the procedure, and/or other parameters.

The control unit 4000 may implement certain automated and/or selectable control features. For example, various routines or programs including operating parameters may be preselected and stored in the control unit 4000 to be selectable by the user, permitting the user to input specified parameters/data to effect appropriate operation of the occlusion treatment device 1000.

The control unit 4000 may control features and/or operation of the occlusion treatment device 1000 based on data or information input by the user. For example, the user may input data related to the occlusion O, such as the dimensions of the occlusion O, the type of tissue comprising the occlusion O, the rate of blood flow within the body lumen L, if any, the volume of blood flow, the percentage of restriction in the body lumen L, the type of body lumen L that is occluded, the location of the body lumen L, the particular dimensions of the body lumen L, the desired advance rate of the occlusion treatment device 1000, the desired position of the cutting tip 204 (for example, spacing of the cutting tip 204 from the inner wall W of the body lumen L and/or the occlusion O, etc.). Based, at least in part, on the data input by the user, the control unit 4000 may calculate and implement automated operating conditions to regulate (e.g., automatically regulate), for example, the position of the cutting tip 204 within the body lumen L by varying the pressure in the expandable members 110_{A}, 110_{B}, as discussed above. Various operating parameters, operating conditions, patient conditions, and the like, may also be recorded and stored within the control unit 4000 to preserve a record of the patient, and/or the details of the procedure.

Referring again to FIGS. 1-4, an exemplary method of treating the occlusion O with the occlusion treatment system 10 includes inserting the outer catheter 100 into the body lumen L and advancing the outer catheter 100 until the distal end portion 104 of the catheter 100 is adjacent the occlusion O. The positioning of the outer catheter 100 may be aided through the use of a guidewire (not shown) that is coaxially positionable within the outer catheter 100. If utilized, a distal end of the guidewire is initially positioned within the body lumen L, e.g., through the use of a needle cannula (not shown). Thereafter, the guidewire can be advanced to a desired location, either adjacent, or beyond, the occlusion O, and a proximal end of the guidewire can be inserted into the distal end 104 of the outer catheter 100 such that the outer catheter 100 can be advanced distally over the guidewire. A dilator/sheath assembly (not shown) may also be used to further facilitate insertion of the outer catheter 100 into the body lumen L.

With the outer catheter 100 positioned in the body lumen L, the expandable members 110_{A}, 110_{B} are expanded into contact with the inner wall W of the body lumen L, and the inner catheter 200 is advanced through the passageway 108 of the outer catheter 100 until the cutting tip 204 is adjacent the occlusion O.

During placement of the outer catheter 100 and the inner catheter 200, the body lumen L and the occlusion O may be visualized using fluoroscopy, or any other suitable method, to facilitate proper positioning or orientation the outer catheter 100 and the inner catheter 200 within the body lumen L.

The cutting tip 204 is utilized to remove material from the occlusion O via longitudinal and/or rotational manipulation of the inner catheter 200, manually and/or via the control unit 4000.

During treatment of the occlusion O, the orientation of the distal end 104 of the outer catheter 100, and thus, the transverse (radial) position of the cutting tip 204 of the inner catheter 200 relative to the central longitudinal axis A, can be altered by manipulating the internal pressure of the expandable members 110_{A}, 110_{B}, either manually or via the control unit 4000.

The ability to transversely displace the cutting tip 204 within the body lumen L in a controllable manner further facilitates symmetrical debulking of the occlusion O, greater debulking of the occlusion O, and improved treatment outcome.

The ability to transversely displace the cutting tip 204 within the body lumen L also enlarges the transverse dimension of the treatment zone beyond that of the cutting tip 204 itself, permitting the use of smaller inner and outer catheters 100, 200, respectively, as compared to those that would otherwise be required to achieve the same debulking capability. By reducing the size of the outer catheter 100 and the inner catheter 200 required to carry out a treatment procedure, patient trauma can also be reduced.

Debulking of the occlusion O continues until the cutting tip 204 of the inner catheter 200 penetrates the occlusion O, during which time, the fluid suction/supply device 3000 (FIG. 1), in combination with the source of fluid 2000, may move fluid from the source of fluid 2000 to the treatment site, for example, for the purpose of aspiration. Additionally, to facilitate the removal of the debris created during debulking of the occlusion O from the body lumen L, the fluid suction/supply device 3000 may create a vacuum force in the vicinity of the cutting tip 204 in order to draw the debris through the occlusion treatment device 1000. By removing debris from the body lumen L, patency of the body lumen L can be increased while maintaining visualization at the site of the occlusion O. Additionally or alternatively, removing debris from the body lumen L can reduce the likelihood of distal embolization of the debris. In some embodiments, the application of a vacuum force by the fluid suction/supply device 3000 allows for the continuous removal of debris, eliminating or reducing the need to remove the inner catheter 200 from the outer catheter 100 to do so and, additionally or alternatively, reducing the overall completion time and complexity of the procedure.

Following penetration of the occlusion O by the cutting tip 204, the inner catheter 200 and/or the outer catheter 100 can be advanced through the occlusion O in order to facilitate further treatment. For example, further treatment can include the placement of a stent, and/or the delivery of a therapeutic agent, such as a thrombolytic agent (e.g., a tissue plasminogen activator (tPA)). To facilitate advancement of the inner catheter 200 and/or the outer catheter 100 through the body lumen L and the occlusion O, one or more components of occlusion treatment device 1000 may include a lubricous coating.

During use of the occlusion treatment system , the positions of the inner catheter 200, the outer catheter 100, and/or the cutting tip 204 may be monitored using a visualization system. For example, the body portion 106 of the outer catheter 100, the body portion 202 of the inner catheter 200, and/or the cutting tip 204 may include one or more markers 220 (FIG. 1), such as radiopaque markers, that can be viewed by the user on a monitor, or the like.

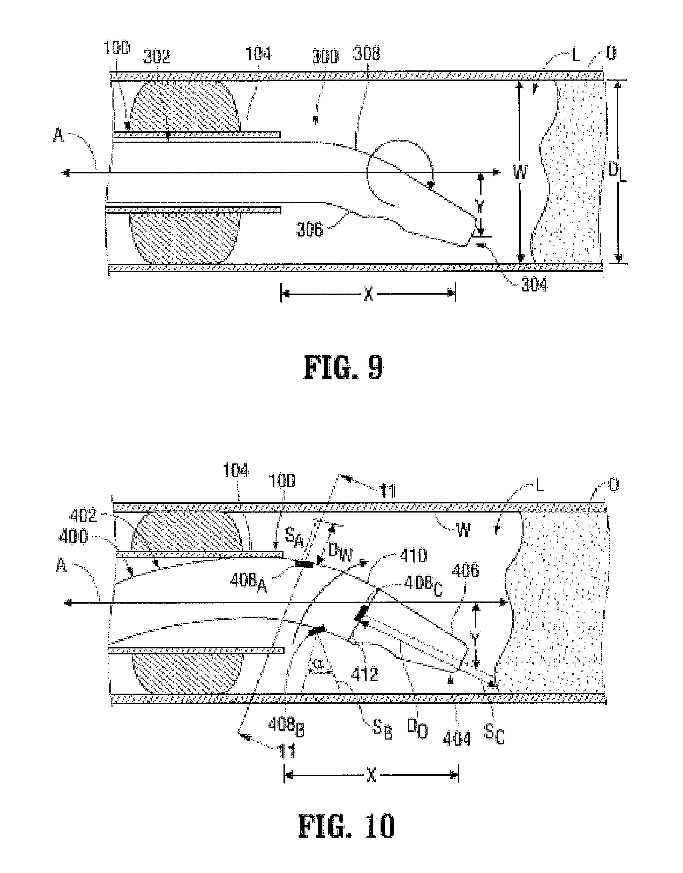

With reference now to FIG. 9, an inner catheter 300 is shown that shares common features with the inner catheter 200 discussed above with respect to FIGS. 1-4, for example, and accordingly, will only be described with respect to differences therefrom.

The inner catheter 300 includes a body portion 302 and a cutting tip 304. The cutting tip 304 is adjacent a distal end 306 of the body portion 302. The body portion 302 of the inner catheter 300 includes a bent portion 308 such that the cutting tip 304 is transversely (radially) offset relative to the central longitudinal axis A of the occlusion treatment device 1000 by a distance Y. The bent portion 308 may define an angle of 15° to 45° with respect to the longitudinal axis A.

The bent portion 308 is disposed along a distal portion of the body portion 302. It should be appreciated, however, that the position of the bent portion 308 may be varied. For example, the bent portion 308 may be located in a central region of the body portion 302, or in a proximal region of the of the body portion 302.

When the inner catheter 300 is in the retracted position, deflection of the cutting tip 304 is limited through engagement of the inner catheter 300 with an inner wall of the outer catheter 100. The inner catheter 300 can be stressed and maintained in a linear, or substantially linear, configuration with the bent portion 308 defining, for example, an angle of 0° to 10° with respect to the longitudinal axis A. As the inner catheter 300 is advanced distally beyond the distal end portion 104 of the outer catheter 100, an axial (longitudinal) distance X is defined between the cutting tip 304 of the inner catheter 300 and the distal end portion 104 of the outer catheter 100.

As the distance X increases due to distal advancement of the inner catheter 300 distally through the outer catheter 100, the stress applied to the inner catheter 300 by the outer catheter 100 is reduced, permitting the inner catheter 300 to assume a non-linear configuration. As can be appreciated through reference to FIG. 9, for example, when the inner catheter 300 is in the non-linear configuration, the distal end portion 306 of the body portion 302, and thus, the cutting tip 304, is deflected away from the central longitudinal axis A of the occlusion treatment device 1000, toward the inner wall W of the body lumen L. As the inner catheter 300 is moved from the substantially linear configuration to the non-linear configuration seen in FIG. 9, the distance Y is increased, which can facilitate greater debulking of the occlusion O, as will be discussed in further detail below.

By contrast, as the distance X is decreased due to retraction of the inner catheter 300 proximally into the outer catheter 100, the stress applied to the inner catheter 300 by the outer catheter 100 is increased, causing the distal end portion 306 of the body portion 302, and thus, the cutting tip 304, to deflect toward the central longitudinal axis A of the occlusion treatment device 1000 away from the inner wall W of the body lumen L, and increasing separation between the cutting tip 304 and the inner wall W of the body lumen L.

Thus, it should be appreciated that, during use of the inner catheter 300, the orientation of the cutting tip 304 can be directly controllable by varying the extent to which the inner catheter 300 is advanced through the outer catheter 100.

Deflection of the cutting tip 304 in relation to the central longitudinal axis A can be limited to a predetermined threshold. For example, the inner catheter 300 can limit the distance Y to a predetermined maximum to facilitate control over the position of the cutting tip 304 to increase the amount of material removed from occlusion O, and/or treatment outcome, thus improving efficacy of the procedure. Specifically, the body portion 302, and the bent portion 308 can be dimensioned such that the distance Y is prevented from exceeding one-half of an internal transverse dimension D_{L} defined by the body lumen L.

Concomitant with axial advancement of the inner catheter 300 through the outer catheter 100, the inner catheter 300 can be rotated, either manually or via the control unit 4000, as discussed above with respect to the inner catheter 200 (FIGS. 1-4), thereby facilitating symmetrical debulking of the occlusion O, and enlarging the transverse dimension of the treatment zone beyond that of the cutting tip 304 itself.

Since the transverse dimension of the treatment zone is governed by axial advancement of the inner catheter 300 and the degree to which the cutting tip 304 is deflected relative to the central longitudinal axis A, rather than by the dimensions of the cutting tip 304 itself, smaller inner and outer catheters 100, 300, respectively, can be utilized to treat the occlusion O, as compared to those that would otherwise be required to achieve the same debulking capability. As a result, patient trauma and complexity of the procedure can be reduced, and more efficacious debulking of the occlusion O can be achieved.

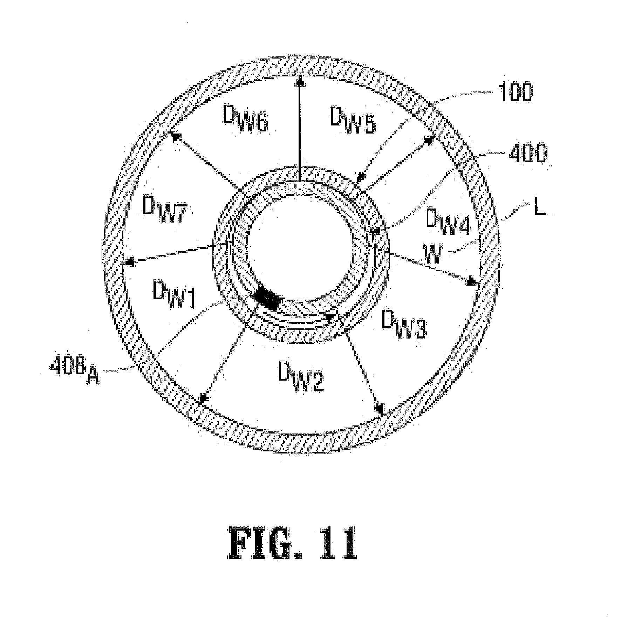

With reference now to FIGS. 10 and 11, an inner catheter 400 is shown that shares common features with the inner catheter 300 discussed above with respect to FIG. 9, and accordingly will only be described with respect to differences therefrom.

The inner catheter 400 includes a body portion 402, a cutting tip 404 that is supported adjacent a distal end portion 406 of the body portion 402, and one or more sensors 408_{A}, 408_{B}, 408_{C} secured to the body portion 402. The sensors 408_{A}, 408_{B}, 408_{C} may measure distance. For example, the sensors 408_{A}, 408_{B}, 408_{C} may be piezoelectric or magnetorestrictive transducers, optical elements, or the like. Additionally or alternatively, the sensors 408_{A}, 408_{B}, 408_{C} may be ultrasonic transceivers such that the ranging

The sensors 408_{A}, 408_{B}, 408_{C} may be secured to an outer sidewall 410 of the body portion 402. For example, sensor(s) 408_{A} may be secured to the outer sidewall 410 of the body portion 402 and oriented to emit a signal S_{A} orthogonally in relation to the outer sidewall 410 (e.g., to measure a distance D_{W} defined between the outer sidewall 410 of the body portion 402 and the inner wall W of the body lumen L). Additionally or alternatively, sensor(s) 408_{B} may be secured to the outer sidewall 410, and oriented to emit a signal S_{B} transversely in relation to the outer sidewall 410 to subtend an acute angle α less than 90°.

The sensor(s) 408_{C} is oriented to emit a signal S_{C} orthogonally in relation to the end wall 412 (e.g., to measure an axial (longitudinal) distance D_{O} defined between the end wall 412 of the body portion 402 and the occlusion O).

The sensors 408_{A}, 408_{B}, and 408_{C} may be positioned at different axial locations along the body portion 402 of the inner catheter 400. In some embodiments, the sensors 408_{A}, 408_{B}, and 408_{C} are positioned at the same axial location along the body portion 402 of the inner catheter 400.

The sensors 408_{A}, 408_{B}, and 408_{C} may be in communication with the control unit 4000. The distances D_{W} and D_{O} may be transmitted to, and acted upon by, the control unit 4000 to continually monitor, and/or regulate, the position of the cutting tip 404 to increase the amount of material removed from occlusion O, and/or treatment outcome. For example, based upon the measured values of D_{W} and D_{O}, the control unit 4000 may modify the extent to which the inner catheter 400 is advanced through the outer catheter 100 and, thus, the distances X and Y, to alter the transverse (radial) position of the cutting tip 404 within the body lumen L. D_{W} and D_{O} can then be re-measured, and the control unit 4000 may again modify the extent to which the inner catheter 400 is advanced through the outer catheter 100 until a desired effect is achieved.

As the inner catheter 400 is advanced distally beyond the distal end portion 104 of the outer catheter 100, ranging is enabled, and the distances D_{W} and D_{O} are measured. With respect to the distance D_{W}, by rotating the inner catheter 400, ranging can be achieved in 360° to account for any irregularities in the body lumen L. For example, the inner catheter 400 may be rotated at 1 Hz, during which time, D_{W} can be repeatedly measured. In some embodiments, the ranging can be ultrasonic ranging based at least in part on echogenicity of the wall inner lining of the body lumen L, connective tissues on the outer wall of the body lumen L, and a thrombus. Additionally or alternatively, ultrasonic ranging may be based at least in part on pulse echo methods.

In the example shown in FIG. 11, D_{W} is measured seven times to calculate distances D_{W1} - D_{W7}, which can be transmitted to, and acted upon by, the control unit 4000 in the manner discussed above. It should be appreciated, however, that the rotational speed of the inner catheter 400, and the number of measurements taken regarding D_{W}, may be altered or varied depending upon the requirements of the particular procedure in which the inner catheter 400 is employed.

Based upon the measured distances D_{W} and D_{O}, the distances X and Y can be varied (e.g., automatically varied by the control unit 4000), based upon predetermined criteria and threshold values, to achieve controlled debulking of the occlusion O. During the course of the procedure, the distances D_{W} and D_{O} can be continually monitored to ensure that the distance D_{W} remains above a predetermined threshold value such that the cutting tip 404 remains a safe distance from the inner wall W of the body lumen L.

Debulking of the occlusion O can continue until the occlusion O is penetrated by the cutting tip 404 in the manner described above.

In the preceding discussion, the sensor(s) 408_{A} is described as measuring the distance D_{W} defined between the outer sidewall 410 of the inner catheter 400 and the inner wall W of the body lumen L, and the sensor(s) 408_{C} is described as measuring the distance D_{O} defined between the end wall 412 of the inner catheter 400 and the occlusion O. During the course of the procedure in which the inner catheter 400 is used, however, as the inner catheter 400 is advanced towards, and through, the occlusion O, it should be understood that each of the sensors 408_{A}, 408_{B}, and 408_{C} may be utilized to calculate either, or both, of the distances D_{W} and D_{O}.

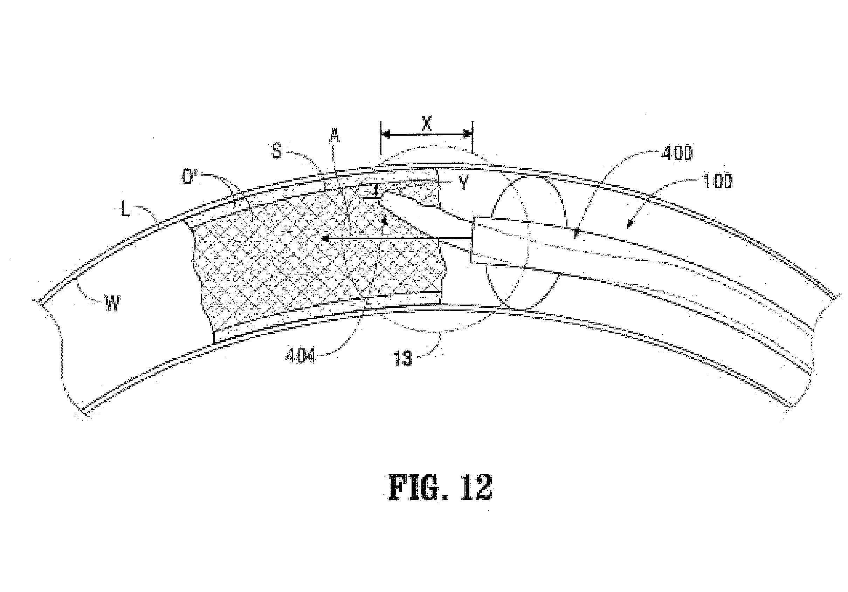

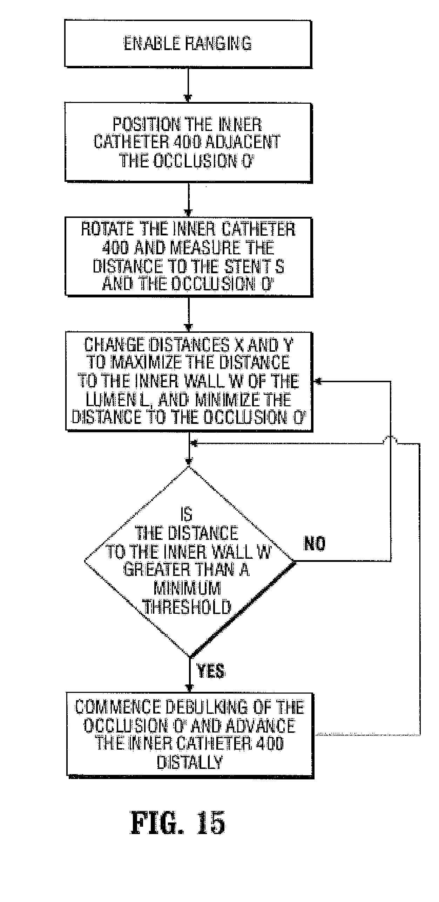

With reference now to FIGS. 12-15, use of the inner catheter 400 will be discussed in connection with the treatment of a restenosed stent S. Generally, stents are placed in a vessel to maintain patency of the vessel. However, over time, an occlusion O' may develop within the stented section of the vessel, occluding the stent S, and causing a restenosis.

Ranging is enabled and the outer catheter 400 is advanced into the volume defined by the stent S. The inner catheter 400 is deployed from the outer catheter 100and is positioned adjacent the occlusion O'.

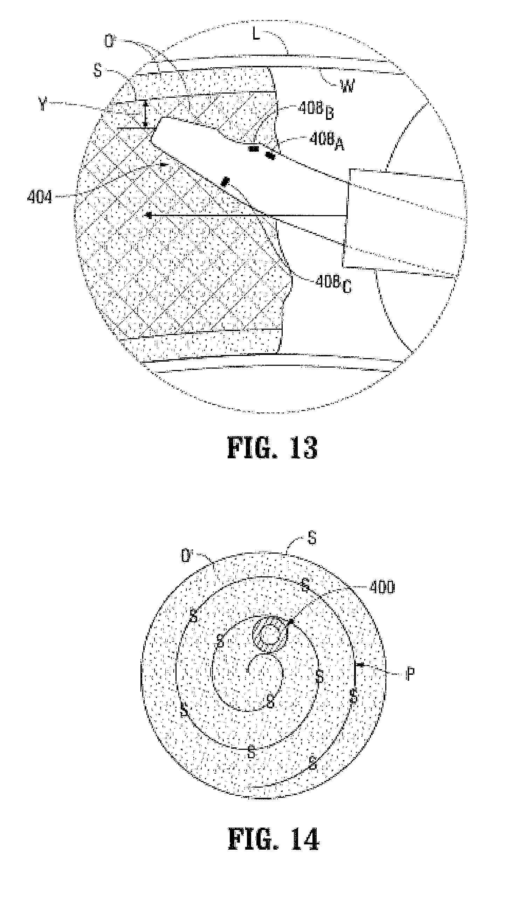

During ranging, due to recognized differences in magnitude between reflections from the material comprising the stent S (e.g., metallic material), and reflections from the tissue including the occlusion O', and the inner wall W of the body lumen L, the location of the stent S in relation to the occlusion O' and the inner wall W of the body lumen L can be determined. As the inner catheter 400 is advanced through the outer catheter 100, the distances X and Y gradually increase, gradually altering the transverse (radial) position of the cutting tip 404 relative to the central longitudinal axis A. Rotation of the inner catheter 400 during advancement of the inner catheter 400 from the outer catheter 100 causes the inner catheter 400 to follow a helical path P (FIG. 14). As the inner catheter 400 follows a helical path, the sensors 408_{A}, 408_{B}, and 408_{C} measure distances to the stent S as well as distances to the occlusion O' and the inner wall W of the body lumen L. The distances measured by the sensors 408_{A}, 408_{B}, and 408_{C} are transmitted to, and acted upon by, the control unit 4000 to determine the precise locations of the stent S, the occlusion O', and the inner wall W of the body lumen L, and, thus, differentiate the stent S from the occlusion O' and the inner wall W of the body lumen L. The control unit 4000 can adjust the distances X and Y, if necessary, and thus, the transverse (radial) position of the cutting tip 404, to maximize the distance to the stent S and/or the inner wall W, and minimize the distance to the occlusion O'.

Once the inner catheter 400 reaches maximum deflection (e.g., when the distance to the stent S reaches a predetermined threshold value) the control unit 4000 may calculate a centroid of the stent S, and the transverse (radial) position of the cutting tip 404 may be adjusted (e.g., the inner catheter 400 can be advanced from, or retracted into, the outer catheter 100) to achieve a desired effect.

With the transverse (radial) position of the cutting tip 404 adjusted, debulking of the occlusion O' by the cutting tip 404 can be commenced. During debulking of the occlusion O', the distances to the stent S, the occlusion O', and the inner wall W of the body lumen L can be monitored. If necessary, the distances X and Y can be varied to facilitate control over the position of the cutting tip 404 to increase the amount of material removed from occlusion O, and achieve maximum debulking of the occlusion O'. For example, the distances X and Y can be varied in an automatic fashion by the control unit 4000 based upon predetermined criteria and threshold values.

Through reference to the foregoing description, it should be understood that the various embodiments of the inner catheter disclosed herein, and the occlusion treatment device, are contemplated as being capable of treating occlusions both mechanically and non-mechanically. For example, each embodiment of the inner catheter described herein may be altered to include one or more ports so as to facilitate the distribution of a treatment agent (e.g. tPA) to the occlusion to increase the efficacy of treatment in the context of occlusions that are more resistant to mechanical debulking. Accordingly, it should be understood that the treatment capabilities of the various embodiments of the occlusion treatment device discussed above may be used alone, or in combination with, other capabilities to effectively remove occlusive material from a body lumen.

Persons skilled in the art will understand that the devices and methods specifically described herein, and illustrated in the accompanying drawings, are non-limiting, exemplary embodiments of the present disclosure, and that the elements and features illustrated or described in connection with one exemplary embodiment may be combined with those of another embodiment without departing from the scope of the present disclosure. For example, it is envisioned that the inner catheter 300 shown in FIG. 9, or the inner catheter 400 shown in FIGS. 10 and 11, may be used in combination with the outer catheter 100 in the manner discussed in connection with FIGS. 1-4.

As well, one skilled in the art will appreciate further features and advantages of the presently disclosed occlusion treatment system based on the above-described embodiments and the claims. Accordingly, the present disclosure is not to be limited by what has been particularly shown and described.

## Claims

1. An occlusion treatment device (1000) comprising:
an outer catheter (100) having a proximal end portion (102) and a distal end portion (104), the outer catheter (100) defining a passageway (108) extending longitudinally therethrough;
at least one expandable member (110_{A},110_{B}) secured to an outer surface (112) of the outer catheter (100); and
an inner catheter (200,300,400) having a body portion (202,302,402) with a proximal end portion and a distal end portion (206,306,406), and the inner catheter (200) including a cutting tip (204,304,404) adjacent the distal end portion (206,306,406) of the body portion (202,302,402), the inner catheter (200,300,400) axially movable along the passageway (108) between a retracted position, in which the inner catheter (200) is stressed within the passageway (108) in a substantially linear orientation, and an advanced position, in which at least a portion of the cutting tip (204,304,404) is exposed from the outer catheter (100), and the inner catheter (200,300,400) assumes a non-linear orientation, wherein advancement and retraction of the inner catheter (200,300,400) relative to the outer catheter (100) transversely moves the cutting tip (204,304,404) relative to a central longitudinal axis defined by the occlusion treatment device (100).

2. The occlusion treatment device (1000) of claim 1, wherein the outer catheter (100) includes first and second expandable members (110_{A},110_{B}) independently expandable relative to one another such that the distal end (104) of the outer catheter (100) is transversely repositionable relative to the central longitudinal axis by varying pressure within the first and second expandable members (110_{A},110_{B}).

3. The occlusion treatment device (1000) of claim 2, wherein the first and second expandable members (110_{A},110_{B}) are positioned equidistant from the distal end (104) of the outer catheter (100).

4. The occlusion treatment device (1000) of claim 2, wherein the first expandable member (110_{A}) is positioned a first distance from the distal end (104) of the outer catheter (100), and the second expandable member (110_{B}) is positioned a second distance from the distal end (104) of the outer catheter (100), the first and second distances being unequal.

5. The occlusion treatment device (1000) of any of claims 2 to 4, wherein the outer catheter (100) further includes a third expandable member (110_{C}) expandable independently of at least one of the first and second expandable members (110_{A},110_{B}).

6. The occlusion treatment device (1000) of any preceding claim, wherein the inner catheter (400) further includes at least one sensor (408_{A},408_{B},408_{C}) oriented to transmit a signal transversely in relation to an outer sidewall (410) of the inner catheter (400) to measure a distance from the sensor (408_{A},408_{B},408_{C}) to an inner wall (W) of a body lumen (L).

7. The occlusion treatment device (1000) of claim 6, wherein the at least one sensor (408_{A}) is oriented to transmit a signal (S_{A}) in orthogonal relation to the outer sidewall (410) of the inner catheter (400).

8. The occlusion treatment device (1000) of claim 6, wherein the inner catheter (400) further includes at least one sensor (408_{C}) oriented to transmit a signal in orthogonal relation to an end wall (412) of the inner catheter (400) to measure a distance to an occlusion (O).

9. The occlusion treatment device (1000) of any of claims 6 to 8, wherein the at least one sensor (408_{A},408_{B},408_{C}) includes a piezoelectric transducer.

10. The occlusion treatment device (1000) of any of claims 6 to 8, wherein the at least one sensor (408_{A},408_{B},408_{C}) includes a magnetorestrictive transducer.

11. The occlusion treatment device (1000) of any of claims 6 to 8, wherein the at least one sensor (408_{A},408_{B},408_{C}) includes an optical element.
